(19) Europäisches Patentamt
European Patent Office
Office européen des brevets

(11) Veröffentlichungsnummer : **0 042 606**
**B1**

(12) # EUROPÄISCHE PATENTSCHRIFT

(45) Veröffentlichungstag der Patentschrift : **13.07.83**

(51) Int. Cl.³ : **C 07 J 3/00**, C 07 J 41/00, C 07 J 63/00

(21) Anmeldenummer : **81104740.6**

(22) Anmeldetag : **20.06.81**

(54) **Verfahren zur Herstellung von 17 Alpha-Hydroxy- und 17a Alpha-Hydroxy-D-homo-etiocarbonsäuren.**

(30) Priorität : **24.06.80 DE 3024008**

(43) Veröffentlichungstag der Anmeldung : **30.12.81 Patentblatt 81/52**

(45) Bekanntmachung des Hinweises auf die Patenterteilung : **13.07.83 Patentblatt 83/28**

(84) Benannte Vertragsstaaten : **AT BE CH DE FR GB IT LI LU NL SE**

(56) Entgegenhaltungen : **HELVETICA CHIMICA ACTA, Band XXI, 1938, Seiten 1317-1326 Basel, CH. K. MIESCHER et al. : « Über Anbau von Seitenketten an t-Dehydroandrosteron »**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Nickolson, Robert, Dr.**
**Fuchsienweg 12C**
**D-1000 Berlin 47 (DE)**
Erfinder : **Kerb, Ulrich, Dr.**
**Prinzregentenstrasse 7**
**D-1000 Berlin 31 (DE)**
Erfinder : **Wiechert, Rudolf, Dr. Prof.**
**Petzowerstrasse 8A**
**D-1000 Berlin 39 (DE)**
Erfinder : **Alig, Leo, Dr.**
**Liebrütistrasse 32**
**Kaiseraugst (CH)**
Erfinder : **Fürst, Andor, Dr.**
**Magnolienpark 14**
**Basel (CH)**
Erfinder : **Müller, Marcel, Dr.**
**Quellenweg 10**
**Frenkendorf (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 17 Alpha-Hydroxy- und 17a Alpha-Hydroxy-D-homo-etiocarbonsäuren

Die Erfindung betrifft den Gegenstand des Anspruchs.

Unter niederen Acylresten sind solche zu verstehen, die sich von physiologisch verträglichen, aliphatischen Carbonsäuren ableiten, wie z. B. Essig-, Propion-, Butter-, Isobutter- und Capronsäure, ableiten.

Nach den Arbeiten von Miescher et al., Helv. *21,* 1317 (1938) könnte man in einem 3-stufigen Verfahren Hydroxy-17-carbonsäuren in der Androstan-Reihe mit der un- natürlichen Konfiguration an C-17 durch Hydratisierung von 17-Cyanhydrin-Derivaten herstellen. Hierzu wird das Carbonnitril in der ersten Stufe mit Chlorwasserstoff/Ethanol behandelt, wobei sich das 3β-Acetoxy-17-hydroxy-5α-chlor-androstan-17-carbonsäureamid bildet.

In der zweiten Stufe wird das so gebildete Hydroxyamid mit methanolischer Kalilauge unter Rückfluß gekocht, wobei man das 3β.17-Dihydroxy-5-androsten-17-carbonsäureamid erhält. In der dritten Stufe wird das Dihydroxiamid mit Natriumpropylat zur freien Carbonsäure hydrolysiert.

Dieses Verfahren hat jedoch den Nachteil, daß die gewünschte Säure nur in sehr geringer Ausbeute erhalten wird. Eigene Versuche, dieses Verfahren zu wiederholen, waren nicht sehr erfolgreich.

Aufgabe der vorliegenden Erfindung war es, ein Verfahren bereitzustellen, daß höhere Ausbeuten liefert und sich mit weniger Reaktionsschritten ausführen läßt.

Die erfindungsgemäße Aufgabe wird dadurch gelöst, daß man die Hydratisierung des Cyanhydrins mit einem Gemisch einer niederen Carbonsäure und ihres Anhydrids in Gegenwart von Perchlorsäure unter Bildung des 3α.17β-Diacyloxy-5-androsten-17β-N-acylcarboxamids bzw. dessen D-Homo-homologen bei Raumtemperatur ausführt und anschließend das so gebildete α-Acylamid in einem mehrwertigen Alkohol mit einem Alkalihydroxid bei höherer Temperatur behandelt.

Der Verlauf der erfindungsgemäßen Reaktion ist insofern überraschend, als man erwartet hätte, daß sich bei Kenntnis der zuvor zitierten Arbeiten von Miescher et al. die Cyanhydrindiacylate, nicht jedoch die Acylamide bilden würden.

Das Verfahren wird so durchgeführt, daß das Gemisch von niederen Alkylsäuren und Alkylsäureanhydriden im Verhältnis von 0,1 : 100, vorzugsweise 1 : 1, und in einer Menge von 1,1-10 Moläq. verwendet wird. Die als saurer Katalysator zugesetzte Perchlorsäure wird in einer Menge von 0,001-0,1, vorzugsweise 0,01 Moläq (bezogen auf Moläq. Steroid) zugesetzt.

Das gebildete Acylamid läßt sich leicht durch Kristallisation abtrennen und in die nächste Reaktionsstufe einsetzen. Hierzu wird das Acylamid in einem mehrwertigen Alkohol wie Äthylenglykol oder Glycerin mit einem Alkalihydroxid wie Kalium- oder Natriumhydroxid längere Zeit erhitzt. Temperaturen über 100 °C sind zweckmäßig, wobei sich die Verwendung von Autoklaven und Schutzgas wie Stickstoff bewährt haben.

Die Umsetzung zur 17-Carbonsäure kann aber auch über die Zwischenstufe des Säureamids geführt werden, wobei das N-Acylcarboxamid mit methanolischer Alkalilauge wie Kalilauge bei Raumtemperatur behandelt wird. Bevorzugt ist jedoch die direkte Umsetzung des N-Acylcarboxamids zur Carbonsäure, ohne das Säureamid zu isolieren.

Die erfindungsgemäß herstellbaren Verbindungen sind Zwischenprodukte zur Herstellung von Corticoiden und antiinflammatorisch wirksame Steroide (s. z. B. DOS 25 38 627, 25 39 595 und 26 14 079).

Die nachfolgenden Beispiele sollen das erfindungsgemäße Verfahren erläutern.

Beispiel 1

Eine Lösung von 3,57 g 3β-Acetoxy-17α-hydroxy-5-androsten-17-carbonitril in 25 ml Eisessig und 25 ml Essigsäureanhydrid wird mit 0,71 ml 70 %iger Perchlorsäure versetzt und das Gemisch 1 Stunde bei 20 °C gerührt. Das Produkt wird in Eiswasser gefällt und mit Methylenchlorid und Essigester extrahiert. Die organischen Phasen werden mit halbgesättigter Natriumhydrogencarbonat-Lösung und anschließend mit Wasser gewaschen. Nach Trocknen über Natriumsulfat wird die Lösung im Vakuum weitgehend eingeengt und der Rückstand aus Methanol kristallisiert. Man erhält 3,41 g 3β.17α-Diacetocy-5-androsten-17-N-acetylcarboxamid vom Schmelzpunkt 208,5-209,5 °C.

A. Eine Lösung von 1,65 g 3β.17α-Diacetoxy-5-androsten-17-N-acetylcarboxamid in 100 ml Methanol wird mit 11 ml In Kaliumhydroxid-Lösung versetzt und das Gemisch 22 Stunden bei Raumtemperatur unter Argon gerührt. Das auskristallisierte Produkt wird abgesaugt und mit 60 %igem Methanol-Wasser gewaschen. Man erhält 1,26 g 3β.17α-Dihydroxy-5-androsten-17β-carbonsäureamid vom Schmelzpunkt 308 °C (Zersetzung).

Eine Lösung von 1,10 g 3β.17α-Dihydroxy-4-androsten-17β-carbonsäureamid in 150 ml Äthylenglykol wird mit einer Lösung von 3,2 g Kaliumhydroxid in 7,0 ml Wasser versetzt und im Rollautoklaven reagieren gelassen. Das Reaktionsgemisch wird auf 20 °C abgekühlt und mit 70 ml Methanol verdünnt. Zur Aufreinigung wird diese Lösung über eine Anionenaustauscher-Säule gegeben und der neutrale Anteil durch Waschen mit 600 ml Methanol abgetrennt. Die Säule wird anschließend mit 80 % Methanol-2n Salzsäure-Lösung eluiert. Die Fraktionen werden vereinigt und das überschüssige Lösungsmittel vorsichtig bei 30 °C im Vakuum eingeengt. Das auskristallisierte Produkt wird abgesaugt, mit 10 %igem Methanol-Wasser-Gemisch gewaschen und getrocknet. Man erhält 920 mg 3β.17α-Dihydroxy-5-androsten-

17-carbonsäure vom Schmelzpunkt 236,5-237,5 °C.

B. Eine Lösung von 500 mg 3β.17α-Diacetoxy-5-androsten-17-N-acetylcarboxamid in 70 ml Äthylenglykol wird mit einer Lösung von 1,2 g Kaliumhydroxid in 3,5 ml Wasser versetzt und das Gemisch 72 Stunden bei 170 °C unter Stickstoff im Rollautoklaven, wie zuvor beschrieben, behandelt und aufgearbeitet. Man erhält 330 mg 3β.17α-Dihydroxy-5-androsten-17-carbonsäure vom Schmelzpunkt 236,5-237 °C.

## Beispiel 2

Eine Lösung von 6,26 g 17α-Hydroxy-17-cyano-4-androsten-3-on in 50 ml Eisessig und 50 ml Essigsäureanhydrid wird mit 0,5 ml 70 %iger Perchlorsäure versetzt und das Gemisch 1 Stunde bei 20 °C stehengelassen. Das Produkt wird in Eiswasser gefällt, abfiltriert und anschließend in 570 ml Methanol gelöst. Zu dieser Lösung wird 61 ml einer ln Kaliumhydroxid-Lösung gegeben und das Gemisch über Nacht bei 20 °C gerührt. Die methanolische Lösung wird mit 500 g Eiswasser verdünnt, das ausgefallene Produkt abgesaugt und getrocknet. Nach mehrmaliger Kristallisation aus Essigester-Methylenchlorid erhält man 5,62 g 17α-Hydroxy-3-oxo-4-androsten-17-carbonsäureamid vom Schmelzpunkt 271-273,5 °C.

Eine Lösung von 500 mg 17α-Hydroxy-3-oxo-4-androsten-17-carbonsäureamid wird analog Beispiel 1 behandelt und aufgearbeitet. Man erhält nach Umkristallisation des Rohproduktes aus Essigester 410 mg 17α-Hydroxy-3-oxo-4-androsten-17-carbonsäure vom Schmelzpunkt 233-234 °C.

## Beispiel 3

Eine Lösung von 2,3 g 3β.17α-Dihydroxy-16α-methyl-5-androsten-17β-carbonitril in 17,5 ml Essigsäureanhydrid und 17,5 ml Essigsäure wird mit 0,18 ml 70 %iger Perchlorsäure versetzt und 1 Stunde bei 25 °C gerührt. Das Produkt wird in Wasser gefällt, abgesaugt, mit Wasser gewaschen und getrocknet. Man erhält 3,10 g 3β.17α-Diacetoxy-16α-methyl-5-androsten-17-N-acetylcarboxamid vom Schmelzpunkt 105,5-108 °C.

Zu einer Lösung von 720 mg 3β.17α-Diacetoxy-16α-methyl-5-androsten-17-N-acetylcarboxamid in 70 ml Äthylenglykol werden 1,8 g Kaliumhydroxid in 5 ml Wasser zugegeben und das Gemisch wird 80 Stunden im Rollautoklaven auf 150 °C erhitzt. Nach Aufarbeitung, wie im Beispiel 1 beschrieben, erhält man 315 mg 3β.17α-Dihydroxy-16α-methyl-5-androsten-17β-carbonsäure vom Schmelzpunkt 258 °C (Zersetzung).

## Beispiel 4

Eine Lösung von 1,20 g 3β-Acetoxy-17α-hydroxy-16β-methyl-5-androsten-17-carbonitril wird analog Beispiel 3 behandelt und aufgearbeitet. Nach Umkristallisation aus Aceton-Essigester erhält man 720 mg 3β.17α-Dihydroxy-16β-methyl-5-androsten-17-carbonsäure vom Schmelzpunkt 214-216 °C.

## Beispiel 5

Eine Suspension von 357 mg 3β-Acetoxy-17α-hydroxy-5-androsten-17β-carbonitril in 5,0 ml Propionsäure und 5,0 ml Propionsäureanhydrid wird mit 0,1 ml 70 %iger Perchlorsäure versetzt und das Gemisch 65 Minuten bei Raumtemperatur reagieren gelassen. Anschließend wird die Lösung in Wasser gefällt und mit Methylenchlorid extrahiert. Die organischen Phasen werden mit Wasser neutral gewaschen, über Natriumsulfat getrocknet und eingeengt. Das so erhaltene Rohprodukt wird über Kieselgel mit einem Methylenchlorid/Aceton-Gradienten chromatographiert und das Produkt aus Methanol umkristallisiert. Man erhält 282 mg 3β-Acetoxy-17α-propionyloxy-5-androsten-17β-N-propionyl-carboxamid vom Schmelzpunkt 96-102 °C.

Dieses Produkt wird analog Beispiel 1A) oder 1B) in 3β.17α-Dihydroxy-5-androsten-17-carbonsäure vom Schmelzpunkt 236-238 °C umgewandelt.

## Beispiel 6

Analog Beispiel 5 werden 357 mg 3β-Acetoxy-17α-hydroxy-5-androsten-17β-carbonitril in 5,0 ml Isobuttersäure und 5,0 ml Isobuttersäureanhydrid mit 0,1 ml 70 %iger Perchlorsäure versetzt und das Gemisch wird 1 Stunde bei Raumtemperatur gerührt. Nach Aufarbeitung und Aufreinigung analog Beispiel 5 erhält man 223 mg 3β-Acetoxy-17α-isobutyryloxy-5-androsten-17β-N-isobutyrylcarboxamid vom Schmelzpunkt 135-142 °C.

Dieses Produkt wird analog Beispiel 1A) oder 1B) in 3β.17α-Dihydroxy-5-androsten-17-carbonsäure vom Schmelzpunkt 237-238,5 °C überführt.

## Beispiel 7

Eine Lösung von 1,0 g 3β-Acetoxy-17aα-hydroxy-D-homo-5-androsten-17aβ-carbonitril (hergestellt analog dem in J. Fried and J. A. Edwards, Organic Reactions in Steroid Chemistry, Van Nostrand

Reinhold Co., N. Y., 1972, Vol. II, beschriebenen Verfahren ; F. 200-205 °C unter Zersetzung) in 7 ml Essigsäure und 7 ml Essigsäureanhydrid wird mit 0,2 ml 70 %iger Perchlorsäure versetzt und 65 Minuten reagieren gelassen. Das Produkt wird in Eiswasser gefällt und abgesaugt. Nach der Chromatographie und Kristallisation aus Hexan erhält man 878 mg 3β.17aα-Diacetoxy-D-homo-5-androsten-17a-N-acetylcarboxamid vom Schmelzpunkt 162-163 °C.

Eine Suspension von 2,08 g 3β.17aα-Diacetoxy-D-homo-5-androsten-17a-N-acetylcarboxamid in 126 ml Methanol wird mit 13,9 ml ln Kaliumhydroxid-Lösung versetzt und 18 Stunden bei 20 °C gerührt. Das ausgefallene Produkt wird anschließend abgesaugt und mit 60 %igem Metanol gewaschen. Man erhält 1,52 g 3β.17aα-Dihydroxy-D-homo-5-androsten-17aβ-carboxamid vom Schmelzpunkt 310-312 °C (Zersetzung).

Eine Lösung von 2,97 g 3β.17aα-Dihydroxy-D-homo-5-androsten-17aβ-carboxamid in 420 ml Ethylenglykol wird mit einer Lösung von 4,8 g Kaliumhydroxid in 21 ml Wasser versetzt und das Gemisch wird 96 Stunden bei 165 °C unter Stickstoff im Rollautoklaven gehalten. Die Reaktionslösung wird auf 20 °C abgekühlt und nach Zugabe von 420 ml 90 %igem Methanol wird das Gemisch über eine Anionenaustauscher-Säule gegeben und der neutrale Anteil mit ca. 4 l 90 %igem Methanol-Wasser-Gemisch abgetrennt. Anschließend wird die Säule mit 80 %iger Methanol-2n Salzsäure-Lösung eluiert. Die Faktionen werden vereinigt und bei 30 °C im Vakuum vorsichtig eingeengt. Das auskristallisierte Produkt wird abgesaugt, mit 10 %igem Methanol-Wasser gewaschen und getrocknet. Man erhält 2,56 g 3β.17aα-Dihydroxy-D-homo-5-androsten-17aβ-carbonsäure vom Schmelzpunkt 248-249 °C (Zersetzung).

Beispiel 8

Eine Lösung von 500 mg 3β-Acetoxy-17aα-hydroxy-D-homo-5-androsten-17aβ-carbonitril in 3,5 ml Essigsäure und 3,5 ml Essigsäureanhydrid wird mit 0,1 ml 70 %iger Perchlorsäure versetzt und 75 Stunden bei 20 °C gerührt. Nach Aufarbeitung, wie im Beispiel 1 beschrieben, wird das Rohprodukt (700 mg) ohne Aufreinigung in 70 ml Ethylenglycol gelöst und mit 1,2 g Kaliumhydroxid in 3,5 ml Wasser versetzt. Die weitere Behandlung und Aufarbeitung erfolgt wie im Beispiel 3 beschrieben. Man erhält 425 mg 3β.17aα-Dihydroxy-D-homo-5-androsten-17aβ-carbonsäure vom Schmelzpunkt 248-249,5 °C (Zersetzung).

**Anspruch**

Verfahren zur Herstellung von 17α-Hydroxy- und 17aα-Hydroxy-D-homo-etiocarbonsäuren der allgemeinen Formel

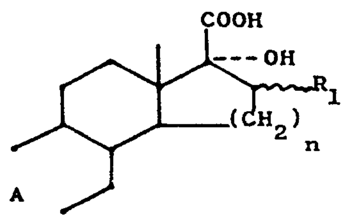

worin

n = 1 oder 2 ist,

A für 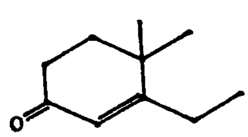 und 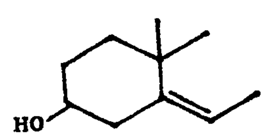

und $R_1$ für H oder $CH_3$ stehen, durch Hydratisierung der entsprechenden Nitrile der allgemeinen Formel

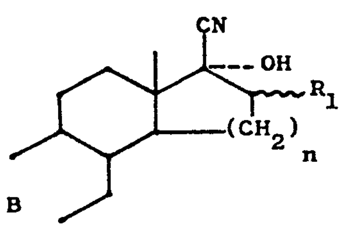

worin

$R_1$ und n die oben angegebene Bedeutung haben und

B für 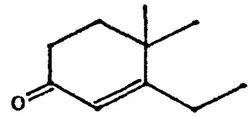 und 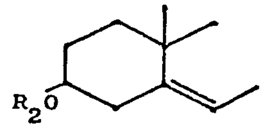

steht, mit $R_2$ in der Bedeutung von Wasserstoff oder eines niederen Acylrestes mit bis zu 6 C-Atomen, dadurch gekennzeichnet, daß man die Hydratisierung des Cyanhydrins mit einem Gemisch aus einer niederen Carbonsäure und ihres Anhydrids in Gegenwart von Perchlorsäure bei Raumtemperatur ausführt und anschließend das gebildete 17β-N-Acylcarboxamid in einem mehrwertigen Alkohol mit einem Alkalihydroxid bei höherer Temperatur behandelt.

**Claim**

**Process for the manufacture of 17α- and 17aα-hydroxy-D-homoetiocarboxylic acids of the general formula**

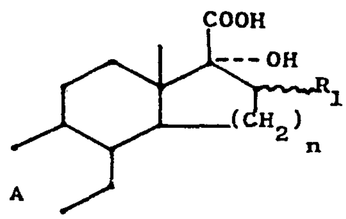

in which

n is 1 or 2,

A represents 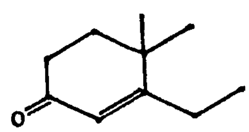 and 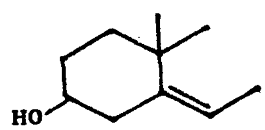

and $R_1$ represents H or $CH_3$ by hydration of the corresponding nitriles of the general formula

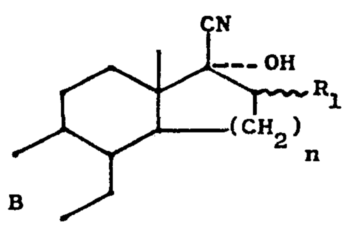

**in wich**

**$R_1$ and n have the meanings given above and**

B represents 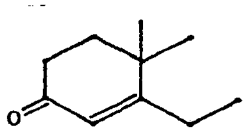 and 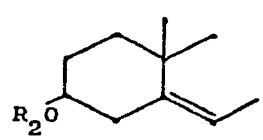

**in which $R_2$ represents hydrogen or a lower acyl radical up to 6 carbon atoms, characterised in that the cyanohydrin is hydrated at room temperature in the presence of perchloric acid using a mixture of a lower carboxylic acid and the anhydride thereof and subsequently the 17β-N-acylcarboxamide formed is treated at a relatively high temperature in a polyhydric alcohol with an alkali metal hydroxide.**

**Revendication**

Procédé de préparation d'acides 17α-hydroxyétiocarboxyliques et d'acides 17aα-hydroxy-D-homo-étiocarboxyliques répondant à la formule générale

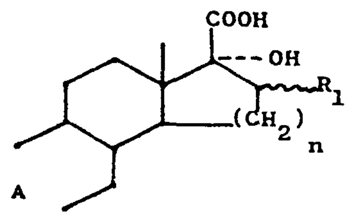

dans laquelle

n est égal à 1 ou à 2,

A représente 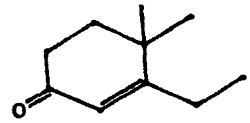 ou 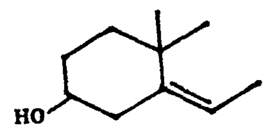

et $R_1$ représente H ou $CH_3$, par hydratation des nitriles correspondants de la formule générale

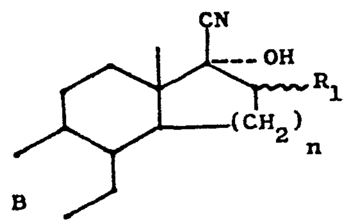

dans laquelle

$R_1$ et n ont la même signification que celle donnée précédemment et

B représente 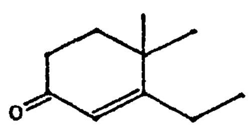 ou 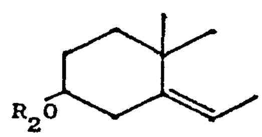

où $R_2$ représente un atome d'hydrogène ou un radical acyle inférieur comprenant jusqu'à 6 atomes de carbone, caractérisé en ce qu'on effectue l'hydratation de la cyanhydrine avec un mélange d'un acide carboxylique inférieur et de son anhydride, en présence d'acide perchlorique, à la température ambiante, et en ce qu'ensuite, à une température supérieure, on traite le 17β-N-acylcarboxamide formé dans un alcool polyfonctionnel avec un hydroxyde de métal alcalin.